# EUROPEAN PATENT SPECIFICATION

(11) **EP 4 436 565 B1**
(45) Date of publication and mention of the grant of the patent: **09.04.2025**
(21) Application number: 23772509.8
(22) Date of filing: 19.09.2023
(51) Int. Cl.: A61K 31/275, A61P 1/16

(54) **THIOACRYLAMIDE DERIVATIVES FOR USE IN THE PREVENTION OR TREATMENT OF LIVER FIBROSIS**
THIOACRYLAMIDDERIVATE ZUR VERWENDUNG BEI DER VORBEUGUNG ODER BEHANDLUNG VON LEBERFIBROSE
DÉRIVÉS DE THIOACRYLAMIDE POUR UTILISATION DANS LA PRÉVENTION OU LE TRAITEMENT DE LA FIBROSE HÉPATIQUE

(30) Priority: 29.11.2022 EP 22210143
(43) Date of publication of application: 02.10.2024
(73) Proprietor: CMR CureDiab Metabolic Research GmbH, 40225 Düsseldorf (DE); Deutsches Diabetes-Zentrum (DDZ) Leibniz-Zentrum für Diabetes-Forschung an der Heinrich-Heine- Universität Düsseldorf, 40225 Düsseldorf (DE)
(72) Inventor: NIERSMANN, Corinna, 47799 Krefeld (DE); ROHBECK, Elisabeth, 40625 Düsseldorf (DE); RODEN, Michael, 40225 Düsseldorf (DE); ECKEL, Jürgen, 40699 Erkrath (DE)
(74) Representative: Eisenführ Speiser
(86) International application number: PCT/EP2023/075745
(87) International publication number: WO 2024/114970

(56) References cited:
- WO-A1-2015/140081
- ROHBECK ELISABETH ET AL: "Positive allosteric [gamma]-aminobutyric acid type A receptor modulation prevents lipotoxicity-induced injury in hepatocytes in vitro", vol. 24, no. 8, 6 June 2022 (2022-06-06), GB, pages 1498 - 1508, XP093034599, ISSN: 1462-8902, Retrieved from the Internet <URL:https://onlinelibrary.wiley.com/doi/full-xml/10.1111/dom.14719> [retrieved on 20230321], DOI: 10.1111/dom.14719
- HEYENS LEEN J. M. ET AL: "Liver Fibrosis in Non-alcoholic Fatty Liver Disease: From Liver Biopsy to Non-invasive Biomarkers in Diagnosis and Treatment", FRONTIERS IN MEDICINE, vol. 8, 14 April 2021 (2021-04-14), XP093094180, Retrieved from the Internet <URL:https://pubmed.ncbi.nlm.nih.gov/33937277/> [retrieved on 20231023], DOI: 10.3389/fmed.2021.615978

## Description

The present invention relates to a thioacrylamide derivative for use in the prevention or treatment of liver fibrosis in a subject or to a pharmaceutical composition comprising such a thioacrylamide derivative for use in the prevention or treatment of liver fibrosis in a subject.

Non-alcoholic fatty liver disease (NAFLD) includes a spectrum of patients with excessive lipid accumulation in the liver. However, in contrast to other situations, such as frequent, excessive alcohol use, there is no defined cause for the increased lipid accumulation in to NAFLD.

Within NAFLD, two groups can be distinguished: non-alcoholic fatty liver (NAFL) and non-alcoholic steatohepatitis (NASH), wherein NASH further includes liver inflammation. For diagnosing NAFLD, hepatic steatosis needs to be confirmed and other causes such as substantial alcohol use, long-term use of a steatogenic medicine, or monogenic hereditary disorders, need to be excluded.

The definition of NASH includes more than 5 % of hepatic steatosis as well as hepatocyte injury and inflammation. Although fibrosis is not required for diagnosing NASH, fibrosis is frequently present in NASH patients, estimates amount to more than 80% of NASH patients. Therefore, NASH patients are frequently divided into subgroups, depending on their fibrosis stage.

NAFL is less dangerous than NASH. Typically, NAFL does not progress to NASH or liver cirrhosis, which is the final stage of many liver diseases. However, in cases NAFLD progresses to NASH, patients may suffer from severe conditions including liver cirrhosis, liver cancer, liver failure or even cardiovascular disease. NASH is more likely to progress to liver cirrhosis or liver cancer. Thus, NASH patients at the end stage usually require a liver transplant.

The numbers of NAFLD patients continuously increase, thus requiring many liver transplants. However, the availability of livers to be transplanted is very limiting Thus, there is a strong need for treating or preventing NAFLD and the underlying conditions.

Moreover, NAFLD not only increases the risk of hepatocellular carcinoma but is also closely linked to cardiovascular diseases and type 2 diabetes mellitus (T2DM). Although several hypoglycemic agents have been widely investigated in the last decades, there is currently no approved medication for the treatment of NAFLD.

Hepatic fibrosis represents a pathological wound-healing process in response to chronic liver injury resulting from non-alcoholic fatty liver disease (NAFLD) / non-alcoholic steatohepatitis (NASH), autoimmune liver diseases, chronic viral infection (hepatitis B virus or hepatitis C virus), alcohol abuse, and metabolic and genetic diseases. The most advanced stage of liver fibrosis is liver cirrhosis, which in turn increases the risk for the development of hepatocellular carcinoma.

Long-lasting liver damage causes the removal of functional hepatic tissue and the formation of fibrotic tissue, which is defined by the excessive accumulation of extracellular matrix (ECM) components such as type I and type III collagens (fibril-forming collagens). During this wound healing process, the ECM proteins are produced by myofibroblasts which in turn come from different sources such as activated hepatic stellate cells (HSC), portal fibroblasts, circulating fibrocytes, bone marrow and epithelial-mesenchymal transition from hepatocytes and cholangiocytes.

However, HSC are widely considered the most relevant source of ECM-producing hepatic myofibroblasts following their transition from a quiescent to a proliferative and fibrogenic myofibroblast-like phenotype. This process, called activation of HSC, can be modulated by multiple signals and pathways such as cytokines, adipocytokines, toll-like receptors, endoplasmic reticulum (ER) stress or autophagy as well as by interactions with other inflammatory and liver resident cells, including hepatocytes, natural killer cells or macrophages.

Although the understanding of the disease epidemiology and pathogenesis continuously progresses and although therapeutic targets can be identified, there is currently no FDA approved therapy for this disease and appropriate therapeutic targets are urgently warranted. Thus, there is a great unmet clinical need for novel therapeutic means that effectively improve or reverse hepatic fibrosis.

Therefore, the primary object of the present invention was to provide a possibility for treating or preventing liver fibrosis in a subject, particularly caused by or resulting from non-alcoholic fatty liver disease (NAFLD) or non-alcoholic steatohepatitis (NASH).

The primary object of the present invention is solved by a thioacrylamide derivative for use in the prevention or treatment of liver fibrosis in a subject,
wherein the thioacrylamide derivative is selected from the group consisting of 2-Cyano-3-cyclopropyl-N-(4-fluoro-3-methyl-phenyl)-3-hydroxy-thioacrylamide (i.e. HK1), 2-Cyano-3-cyclopropyl-3-hydroxy-N-(3-methyl-4-nitro-phenyl)-thioacrylamide (i.e. HK3), a tautomer of HK1, a tautomer of HK3, or mixtures thereof, or pharmaceutically acceptable salts thereof or solvates thereof,
wherein the liver fibrosis is caused by or resulting from non-alcoholic fatty liver disease (NAFLD).

It was surprisingly found that HK1 or HK3 can be applied to treat or prevent liver fibrosis, as set forth herein below, particularly in the example section.

Surprisingly, it was found that HK3 provided a superior effect compared to HK1 in the majority of experiments.

It is thus preferred that the thioacrylamide derivative is HK3.

HK1 and HK3 belong to a family of positive allosteric modulators (PAMs) of the γ aminobutyric acid type A (GABAA) receptor with exclusive peripheral action.

Preferably, HK1 refers to the compound according to the following structure:

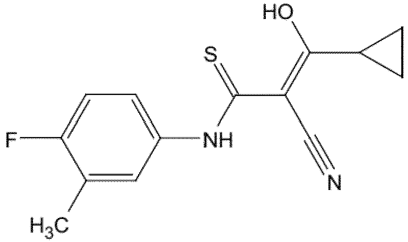

Preferably, HK3 refers to the compound according to the following structure:

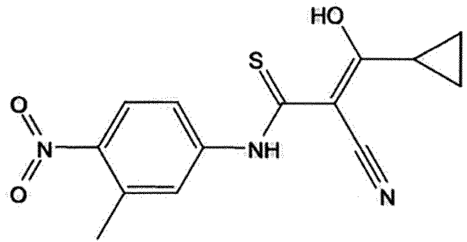

The term "tautomer", as used herein, preferably refers to the keto-enol-tautomer of a structure. Thus, preferably, the term "tautomer of HK1", as used herein, refers to the structure of HK1, as shown above, wherein the C=C-OH group next to the cyclopropyl group is a C-C=O group. Likewise, the term "tautomer of HK3", as used herein, preferably to the structure of HK3, as shown above, wherein the C=C-OH group next to the cyclopropyl group is a C-C=O group.

Preferably, the term "pharmaceutically acceptable salt", as used herein, refers to non-toxic salts. Preferably, the salts are derived from acids such as acetic acid, aconitic acid, citric acid, embolic acid, enanthic acid, hydrobromic acid, hydrochloric acid, lactic acid, maleic acid, malic acid, methanesulphonic acid, phosphoric acid, phthalic acid, salicylic acid, sorbic acid, succinic acid, sulfuric acid or tartaric acid.

Preferably, the term "solvate", as used herein, refers to a compound or salt thereof, further including a stoichiometric or non-stoichiometric amount of solvent bound by non-covalent intermolecular forces, e.g. a hydrate in case the solvent is water.

Preferably, a liver fibrosis caused by or resulting from NAFLD refers to cases, in which liver fibrosis is diagnosed in a patient suffering from NAFLD, e.g. a NAFL or NASH patient.

The present invention further relates to a pharmaceutical composition comprising a thioacrylamide derivative according to the invention for use in the prevention or treatment of liver fibrosis in a subject,
wherein the liver fibrosis is caused by or resulting from non-alcoholic fatty liver disease (NAFLD).

As described herein, HSCs are considered the most relevant source of ECM-producing hepatic myofibroblasts, after the HSCs are activated. It is thus preferred for the thioacrylamide derivative or pharmaceutical composition for use according to the invention that the liver fibrosis is caused by or characterized by or includes the activation of hepatic stellate cells.

Preferably, the term "hepatic stellate cells", as used herein, refers to human hepatic stellate cells.

In a healthy state, HSCs are present as quiescent cells. However, in the case of liver fibrosis, HSCs are activated. The activated state is typically characterized by proliferation, production of extracellular matrix proteins, contractility, and chemotaxis. Further, the amount of stored vitamin A in HSCs decreases progressively in the activated state and activated HSCs may secrete collagen, thus contributing to liver fibrosis. Activated HSCs may be identified by immunofluorescent analyses staining collagen on the cell surface with a Sirius Red staining.

The term "liver fibrosis", as used herein, refers to the excessive accumulation of extracellular matrix proteins due to chronic liver diseases. Preferably, liver fibrosis refers to the accumulation of alpha-1 type 1 collagen and/or fibronectin in the hepatic tissue.

It was surprisingly found that HK1 and HK3 can reduce or revert the upregulation of the pro-fibrotic proteins alpha-1 type 1 collagen and fibronectin, which occurs during fibrosis. It is thus preferred for the thioacrylamide derivative or pharmaceutical composition for use according to the invention that the liver fibrosis is caused by or characterized by or includes increased amounts of alpha-1 type 1 collagen and/or fibronectin in the hepatic tissue of the subject.

Furthermore, an increased secretion of leukemia inhibitory factor (LIF), vascular endothelial growth factor A (VEGFA), Tumor necrosis factor α (TNF-α), interleukin-6 (IL-6) and interleukin-8 (IL-8) by HSCs was observed in liver fibrosis. Surprisingly, it was found that HK1 and HK3 can reduce or revert this upregulation of the leukemia inhibitory factor (LIF), vascular endothelial growth factor A (VEGFA), Tumor necrosis factor α (TNF-α), interleukin-6 (IL-6) and interleukin-8 (IL-8).

Thus, it is preferred for the thioacrylamide derivative or pharmaceutical composition for use according to the invention that the liver fibrosis is caused by or characterized by or includes increased amounts of one, two, three or all proteins selected from the group consisting of leukaemia inhibitory factor (LIF), vascular endothelial growth factor A (VEGFA), Tumor necrosis factor α (TNF-α), interleukin-6 (IL-6) and interleukin-8 (IL-8) in the blood, preferably in the blood serum, of the subject.

It was further found that the levels of TGF-β1 were increased in liver fibrosis. Particularly, it was found that TGF-β1 stimulation caused an increase of alpha-1 type 1 collagen, fibronectin, leukaemia inhibitory factor (LIF), vascular endothelial growth factor A (VEGFA) and interleukin-6 (IL-6) in in cultured hepatic stellate cells.

It is thus preferred for the thioacrylamide derivative or pharmaceutical composition for use according to the invention that the increased amounts of the, one, two, three or more or all proteins are caused by increased protein amounts of transforming growth factor β1 (TGF-β1),
preferably in the hepatic tissue of the subject,
preferably wherein TGF-β1 refers to activated TGF-β1.

Typically, TGF-β is secreted by many cell types, including macrophages, in a latent form in which it is complexed with two other polypeptides, latent TGF-beta binding protein (LTBP) and latency-associated peptide (LAP).

TGF-β1 is synthesized as precursor molecule containing a propeptide region in addition to the TGF-β1 homodimer. After it is synthesized, the TGF-β1 homodimer interacts with a Latency-Associated Peptide (LAP), a protein derived from the N-terminal region of the TGF-β1 gene product, forming a complex called Small Latent Complex (SLC). This complex remains in the cell until it is bound by another protein called Latent TGF-β-Binding Protein (LTBP), forming a larger complex called Large Latent Complex (LLC). It is this LLC that gets secreted to the extracellular matrix (ECM).

In most cases, before the LLC is secreted, the TGF-β1 precursor is cleaved from the propeptide but remains attached to it by noncovalent bonds. After its secretion, it remains in the extracellular matrix as an inactivated complex containing both the LTBP and the LAP, which need to be further processed in order to release active TGF-β1. The attachment of TGF-β1 to the LTBP is by disulfide bonds, which allows it to remain inactive by preventing it from binding to its receptors.

Several possibilities, such as serum proteinases, integrins, pH changes, reactive oxygen species or further factors may promote the activation of TGF- β1 and thus the release of active TGF-β from the complex.

Furthermore, as described above, the most advanced stage of liver fibrosis is liver cirrhosis, which in turn increases the risk for the development of hepatocellular carcinoma. Liver cirrhosis thus requires treatment or needs to be prevented.

Thus, it is preferred for the thioacrylamide derivative or pharmaceutical composition for use according to the invention that the prevention or treatment of liver fibrosis is or includes the prevention or treatment of liver cirrhosis.

It has been found that already low amounts of HK1 or HK3 are sufficient for the prevention or treatment of liver fibrosis. It is thus preferred for the thioacrylamide derivative or pharmaceutical composition for use according to the invention that the prevention or treatment comprises the administration of at least 1 mg/kg body weight of the subject, preferably at least 2.5 mg/kg, preferably at least 5 mg/kg, preferably at least 7.5 mg/kg, preferably at least 10 mg/kg, of HK1, of HK3 or of a combination of HK1 and HK3.

It is further preferred for the thioacrylamide derivative or pharmaceutical composition for use according to the invention that the prevention ortreatment comprises the administration of a daily dose of at least 1 mg/kg body weight of the subject, preferably at least 2.5 mg/kg, preferably at least 5 mg/kg, preferably at least 7.5 mg/kg, preferably at least 10 mg/kg, of HK1, of HK3 or of a combination of HK1 and HK3.

Preferably, the term "daily dose", as used herein, refers to the total dose of the thioacrylamide derivative(s) selected from the group consisting of 2-Cyano-3-cyclopropyl-N-(4-fluoro-3-methyl-phenyl)-3-hydroxy-thioacrylamide (HK1), 2-Cyano-3-cyclopropyl-3-hydroxy-N-(3-methyl-4-nitro-phenyl)-thioacrylamide (HK3), a tautomer of HK1, a tautomer of HK3, or mixtures thereof, or pharmaceutically acceptable salts thereof or solvates thereof.

Further preferably, the term "daily dose", as used herein, refers to an average daily amount per body weight of the thioacrylamide derivative(s) selected from the group consisting of 2-Cyano-3-cyclopropyl-N-(4-fluoro-3-methyl-phenyl)-3-hydroxy-thioacrylamide (HK1), 2-Cyano-3-cyclopropyl-3-hydroxy-N-(3-methyl-4-nitro-phenyl)-thioacrylamide (HK3), a tautomer of HK1, a tautomer of HK3, or mixtures thereof, or pharmaceutically acceptable salts thereof or solvates thereof, which is administered to a subject and does not necessarily require the administration to be performed every single day of a given time period. If the administration of the thioacrylamide derivative(s) is performed once or more in a given number of days, the daily dose can be calculated by dividing the total administered amount of the thioacrylamide derivative(s) per body weight of the subject by the respective given number of days.

It is further preferred for the thioacrylamide derivative or pharmaceutical composition for use according to the invention that the administration is performed at an administration scheme of at least once in 7 days, preferably at least once in 6 days, preferably at least once in 5 days, preferably at least once in 4 days, preferably at least once in 3 days, preferably at least once in 2 days, preferably at least once a day, preferably at least twice a day, preferably at least three times a day, preferably at least four times a day, preferably at least five times a day.

Preferably, the administration scheme is applied over a time period of at least 1 week, preferably at least 2 weeks, preferably at least 3 weeks, preferably at least 4 weeks, preferably at least 6 weeks, preferably at least 2 months, preferably at least 3 months, preferably at least 4 months, preferably at least 5 months, preferably at least 6 months, preferably at least 7 months, preferably at least 8 months, preferably at least 9 months, preferably at least 10 months, preferably at least 11 months, preferably at least 12 months, preferably at least 15 months, preferably at least 18 months, preferably at least 24 months.

Applying the administration scheme for a certain time period as described herein and within a certain regularity as described herein are not mutually exclusive. E.g., an administration of at least once in 7 days for at least 4 weeks describes the regularity of once in 7 days for a duration of at least 4 weeks or longer. The term "once in 7 days", as used herein, is to be understood as one administration within a time frame of 7 days, wherein the administration may be at the beginning, the end or at any other time point within the 7 days. In case the administration is repeated, i.e. the time period of the administration scheme is longer than the administration interval, the time point of the respective administration (the beginning, the end or at any other time point within the interval) may vary between the respective administration intervals. As an example, in an administration of at least once in 7 days for at least 4 weeks, the administration in the first 7 days (first interval) may be performed at the beginning (day 1) of the interval and the administration in the second 7 days (second interval) may be performed on day 3 of the interval. The same applies accordingly to any other regularity and time period as described herein.

Preferably, the administration is performed by or comprises an administration via an administration route selected from the group consisting of oral, nasal, sub-lingual, intravenous, intra-arterial, subcutaneous, inhalation, and absorption over a mucous membrane.

Preferably, in case the liver fibrosis, as described herein, is caused by or characterized by or includes a characteristic, the treatment or prevention provides a reduction or prevention of this characteristic.

The present invention further relates to a pharmaceutical composition comprising one or more thioacrylamide derivatives,
wherein the, one or more or all thioacrylamide derivative is/are selected from the group consisting of 2-Cyano-3-cyclopropyl-N-(4-fluoro-3-methyl-phenyl)-3-hydroxy-thioacrylamide (HK1), 2-Cyano-3-cyclopropyl-3-hydroxy-N-(3-methyl-4-nitro-phenyl)-thioacrylamide (HK3), a tautomer of HK1, a tautomer of HK3, or mixtures thereof, or pharmaceutically acceptable salts thereof or solvates thereof,
wherein the amount of the one or more thioacrylamide derivative(s) is sufficient for preventing or treating liver fibrosis in a subject,
wherein the liver fibrosis is caused by or resulting from non-alcoholic fatty liver disease (NAFLD).

It is preferred that the thioacrylamide derivative is HK3.

Preferably, the pharmaceutical composition according to the invention further comprises one or more substances selected from stabilizers, binders, fillers, disintegrants, and lubricants.

Preferably, the pharmaceutical composition according to the invention is present in a form selected from the group consisting of pills, tablets, lozenges, granules, capsules, preferably hard or soft gelatine capsules, aqueous solutions, alcoholic solutions, oily solutions, syrups, emulsions, suspensions, suppositories, pastilles, solutions for injection or infusion, ointments, tinctures, creams, lotions, powders, sprays, transdermal therapeutic systems, nasal sprays, aerosols, aerosol mixtures, microcapsules, implants, rods, patches and gels.

Preferably, the amount of the one or more thioacrylamide derivative(s) is sufficient for preventing or treating, as described herein, refers to the total amount of the thioacrylamide derivative(s) selected from the group consisting of 2-Cyano-3-cyclopropyl-N-(4-fluoro-3-methyl-phenyl)-3-hydroxy-thioacrylamide (HK1), 2-Cyano-3-cyclopropyl-3-hydroxy-N-(3-methyl-4-nitro-phenyl)-thioacrylamide (HK3), a tautomer of HK1, a tautomer of HK3, or mixtures thereof, or pharmaceutically acceptable salts thereof or solvates thereof.

Further preferably, the amount of the one or more thioacrylamide derivative(s) is sufficient for preventing or treating, as described herein, refers to an amount in the range of from 5 mg to 10 g, preferably in the range of from 10 mg to 5 g, preferably in the range of from 50 mg to 2.5 g, preferably in the range of from 100 mg to 1.5 g, of the one or more thioacrylamide derivative(s).

It is further preferred that the amount sufficient for preventing or treating liver fibrosis refers to liver fibrosis caused by or characterized by or including the activation of hepatic stellate cells. Thus, it is preferred for the pharmaceutical composition according to the invention that the liver fibrosis is caused by or characterized by or includes the activation of hepatic stellate cells.

It is further preferred that the amount sufficient for preventing or treating liver fibrosis refers to liver fibrosis caused by or characterized by or including increased amounts of alpha-1 type 1 collagen and/or fibronectin in the hepatic tissue of the subject. Thus, it is preferred for the pharmaceutical composition according to the invention that the liver fibrosis is caused by or characterized by or includes increased amounts of alpha-1 type 1 collagen and/or fibronectin in the hepatic tissue of the subject. Preferably, the increased amounts of the, one, two, three or more or all these proteins are caused by increased protein amounts of transforming growth factor β1 (TGF-β1), preferably in the hepatic tissue of the subject, preferably wherein TGF-β1 refers to activated TGF-β1.

It is further preferred that the amount sufficient for preventing or treating liver fibrosis refers to liver fibrosis caused by or characterized by or including increased amounts of one or two or all proteins selected from the group consisting of leukaemia inhibitory factor (LIF), vascular endothelial growth factor A (VEGFA) and interleukin-6 (IL-6) in the blood, preferably in the blood serum, of the subject. Thus, it is preferred for the pharmaceutical composition according to the invention that the liver fibrosis is caused by or characterized by or includes increased amounts of one or two or all proteins selected from the group consisting of leukaemia inhibitory factor (LIF), vascular endothelial growth factor A (VEGFA) and interleukin-6 (IL-6) in the blood, preferably in the blood serum, of the subject. Preferably, the increased amounts of the, one, two, three or more or all these proteins are caused by increased protein amounts of transforming growth factor β1 (TGF-β1), preferably in the hepatic tissue of the subject, preferably wherein TGF-β1 refers to activated TGF-β1.

It is further preferred that the amount sufficient for preventing or treating liver fibrosis refers to liver fibrosis, which has advanced to liver cirrhosis. Thus, it is preferred for the pharmaceutical composition according to the invention that the prevention or treatment of liver fibrosis is or includes the prevention or treatment of liver cirrhosis.

What has been said herein with regard to the thioacrylamide derivative or pharmaceutical composition for use according to the invention, applies accordingly to the pharmaceutical composition according to the invention.

Furthermore, the invention relates to a thioacrylamide derivative or pharmaceutical composition for use according to the invention for the treatment or prevention of NASH, wherein the treatment or prevention includes a reduction of the apoptosis of hepatocytes, preferably wherein the apoptosis is caspase 3/7-mediated apoptosis.

It is known that the hepatocytes of NASH patients present increased NF-κB activity, which finally results in increased hepatocyte apoptosis, wherein the apoptosis is mediated by caspase 3/7.

It was surprisingly found that HK1 and HK3 were able to reduce or even prevent the caspase 3/7-mediated apoptosis, as shown in Example 4. Thus, HK1 and HK3 may be used to treat or prevent NASH, wherein the treatment or prevention includes a reduction of the apoptosis of hepatocytes, as described herein. Interestingly, it was found that some other thioacrylamide derivatives were not able to provide such an effect. Furthermore, it was surprisingly found that HK1 and HK3, particularly HK3, provided a stronger effect than other thioacrylamide derivatives, a shown in Example 5.

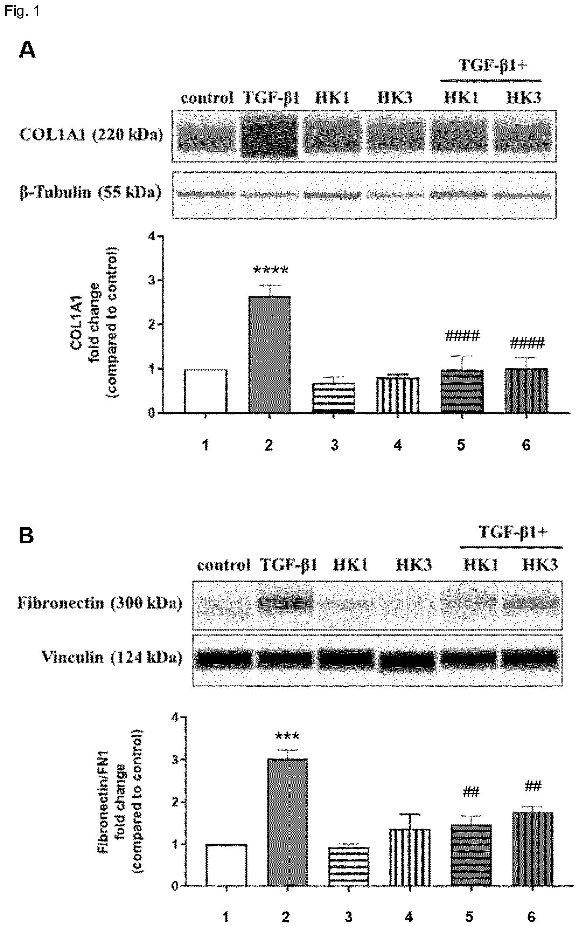

Fig. 1 shows the results of Example 1, i.e. the protein levels of alpha-1 type 1 collagen (A) and of fibronectin (B) obtained by Western blotting, with the untreated control (1), cells treated with TGF-β1 (2), cells treated with HK1 (3), cells treated with HK3 (4), cells treated with TGF-β1 and HK1 (5), cells treated with TGF-β1 and HK3, with ***: p<0.001 and ****: p<0.0001 versus control (1) and with ##: p<0.01 and ####: p<0.0001 versus TGF-β1 (2).

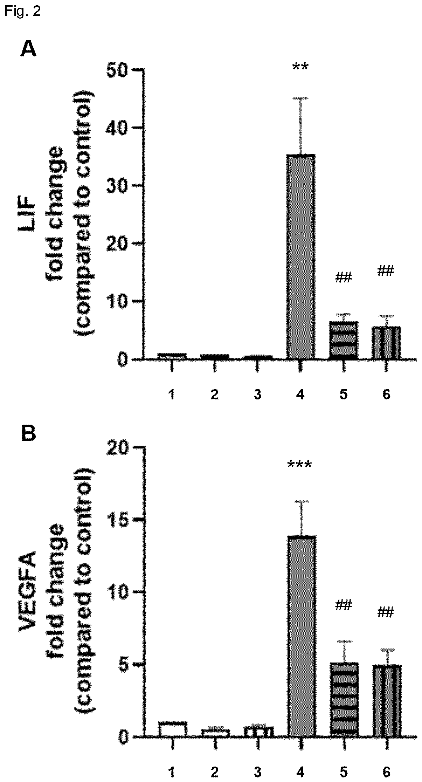

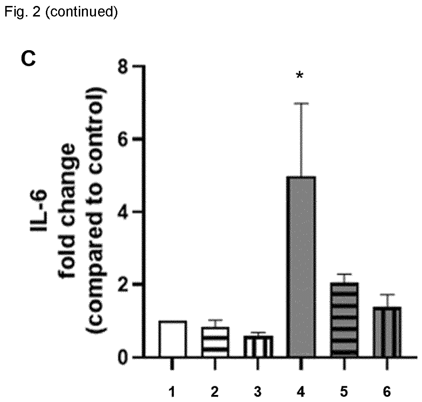

Fig. 2 shows the results of Example 2, i.e. the protein levels of LIF (A) of VEGFA (B) and of IL-6 (C), with the untreated control (1), cells treated with HK1 (2), cells treated with HK3 (3), cells treated with TGF-β1 (4), cells treated with TGF-β1 and HK1 (5), cells treated with TGF-β1 and HK3 (6), with *: p<0.05, **: p<0.01, and ***: p<0.001 versus control (1) and with ##: p<0.01 versus TGF-β1 (2).

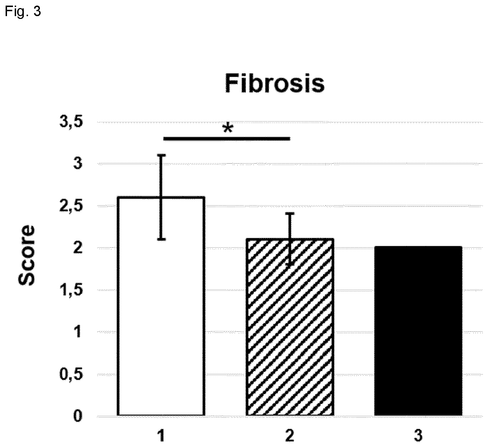

Fig. 3 shows the results of Example 3, i.e. the fibrosis score, with the vehicle treated CCl₄ control (1), mice treated with CCl₄ and HK3 (2) and, baseline mice without a further CCl₄ treatment (3), with *: p<0.05.

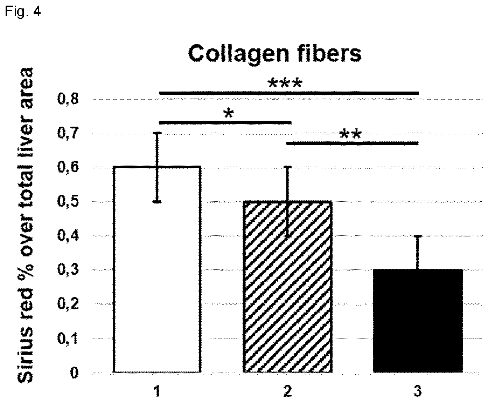

Fig. 4 shows the results of Example 3, i.e. the amount of collagen fibers measured by Sirius red staining, with the vehicle treated CCl₄ control (1), mice treated with CCl₄ and HK3 (2) and, baseline mice without a treatment (3), with *: p<0.05, **: p<0.01 and ***: p<0.001.

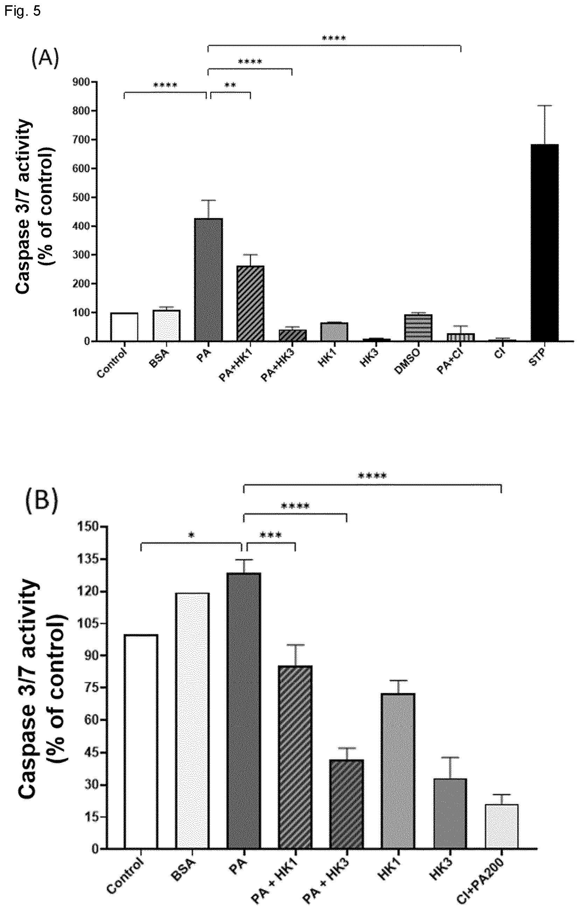

Fig. 5 shows the results of Example 4, i.e. the Caspase 3/7 activity in HepG2 cells (A), with the untreated control (Control), the vehicle treated control (BSA), palmitate treatment (PA), a combined treatment of palmitate and HK1 (PA+HK1), a combined treatment of palmitate and HK3 (PA+HK3), a treatment with HK1 (HK1), a treatment with HK3 (HK3), the vehicle treated control (DMSO), a combined treatment of palmitate and the Caspase 3/7 inhibitor (PA+CI), a treatment with the Caspase 3/7 inhibitor (CI) and the treatment with Staurosporine as positive control (STP), or in primary human hepatocytes (B), with the untreated control (Control), the vehicle treated control (BSA), palmitate treatment (PA), a combined treatment of palmitate and HK1 (PA+HK1), a combined treatment of palmitate and HK3 (PA+HK3), a treatment with HK1 (HK1), a treatment with HK3 (HK3), a combined treatment of palmitate and the Caspase 3/7 inhibitor (CI+PA200), with *: p≤0.05, **: p≤0.01, ***: p≤0.001 and ****: p≤0.0001.

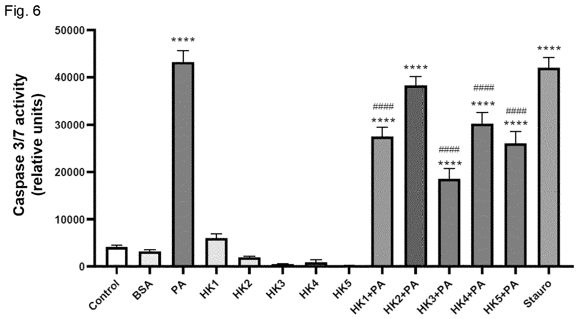

Fig. 6 shows the results of Example 5, i.e. the Caspase 3/7 activity in HepG2 cells, with the untreated control (Control), the vehicle treated control (BSA), palmitate treatment (PA), a treatment with HK1 (HK1), a treatment with HK2 (HK2), a treatment with HK3 (HK3), a treatment with HK4 (HK4), a treatment with HK5 (HK5), a combined treatment of palmitate and HK1 (HK1+PA), a combined treatment of palmitate and HK2 (HK2+PA), a combined treatment of palmitate and HK3 (HK3+PA), a combined treatment of palmitate and HK4 (HK4+PA), a combined treatment of palmitate and HK5 (HK5+PA), and the treatment with Staurosporine as positive control (Stauro), with ****: p≤0.0001 compared to control and ####: p≤0.0001 comparing the combined treatment (palmitate and the respective thioacrylamide derivative) with the palmitate treatment (PA).

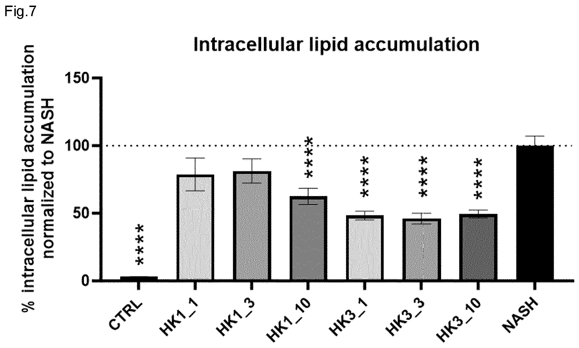

Fig. 7 shows the results of Example 6.2, i.e. the intracellular lipid accumulation with the negative control (CTRL), a treatment with 1 µM HK1 (HK1_1), a treatment with 3 µM HK1 (HK1_3), a treatment with 10 µM HK1 (HK1_10), a treatment with 1 µM HK3 (HK3_1), a treatment with 3 µM HK3 (HK3_3), a treatment with 10 µM HK3 (HK3_10) and the positive control (NASH), average values are shown compared to the NASH control at the same time point, error bars indicate SEM, with **** p<0.0001 for a significant decrease in lipid accumulation compared to NASH, n=16 spheroids per measurement.

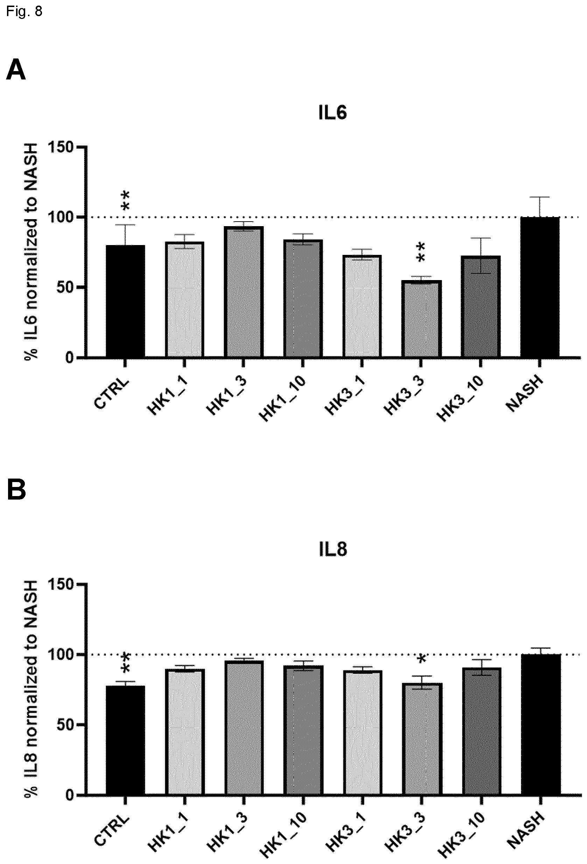

Fig. 8 shows the results of Example 6.3, i.e. the secretion of IL-6 (A) and of IL-8 (B), with the negative control (Ctrl), a treatment with 1 µM HK1 (HK1_1), a treatment with 3 µM HK1 (HK1_3), a treatment with 10 µM HK1 (HK1_10), a treatment with 1 µM HK3 (HK3_1), a treatment with 3 µM HK3 (HK3_3), a treatment with 10 µM HK3 (HK3_10) and the positive control (NASH), average values are shown compared to the NASH control at the same time point, error bars indicate SEM, * p<0.05; ** p<0.01 for a significant decrease in cytokine levels compared to NASH, n=5 with 80 spheroids per measurement.

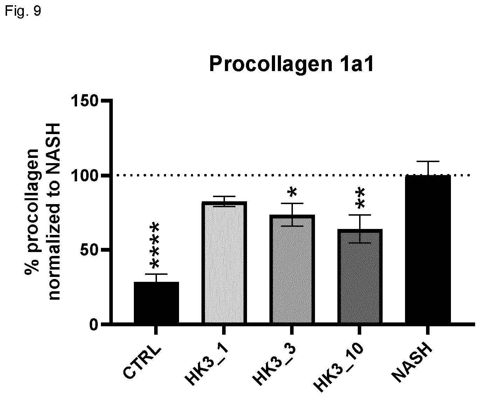

Fig. 9 shows the results of Example 6.4, i.e. the fibrosis assessment, with the negative control (Ctrl), a treatment with 1 µM HK3 (HK3_1), a treatment with 3 µM HK3 (HK3_3), a treatment with 10 µM HK3 (HK3_10) and the positive control (NASH), average values are shown compared to the NASH control at the same time point, error bars indicate SEM. * p<0.05; ** p<0.01; **** p<0.0001 for a significant decrease in procollagen 1a1 levels compared to NASH, n=5 with 80 spheroids per measurement.

Further aspects and advantages of the invention result from the subsequent description of preferred examples.

### Examples

### Example 1: Prevention of the upregulation of pro-fibrotic proteins

The human hepatic stellate cell line LX2 was purchased from Merck. The cells were used up to passage ten and incubated under sterile conditions at 37 °C and 5% CO2. The cells were cultivated in medium containing Dulbecco's Modified Eagle's Medium with 4.5 g/l glucose (ThermoFisher), 4 mmol/I glutamine (ThermoFisher), 2% fetal bovine serum (FBS) (Merck), 100 U/ml penicillin and 100 µg/ml streptomycin (Merck). After 2 days, LX2 cells were starved for FBS for 24 h and then pretreated with 10 µM HK1 (Taros Chemicals GmbH & Co. KG) or 10 µM HK3 (Taros Chemicals GmbH & Co. KG) for 30 min. Subsequently, 5 ng/ml recombinant TGF-β1 (R&D Systems, Minneapolis, MN, USA) was added to the hepatic stellate cell cultures for 24 h. LX2 cells were also incubated with 5 ng/ml TGF-β1 alone as positive control for the hepatic stellate cell activation. Recombinant TGF-β1 was dissolved in sterile 4 mmol/I HCl (Merck) containing 0.1% bovine serum albumin (Roth Karlsruhe, Germany) as carrier protein. HK1 and HK3 were dissolved in sterile dimethyl sulfoxide (DMSO) (Sigma-Aldrich, Munich, Germany). Stimulations with 10 µM HK1, 10 µM HK3 and the corresponding vehicles (HCl and DMSO with or without HCl) alone for 24 h were also included as treatments.

For the measurement of the pro-fibrotic proteins, Western Blotting was performed. Proteins were extracted from whole cell lysate of LX2 cells using RIPA lysis buffer containing 50 mmol/I Tris-HCl (pH 8.0), 150 mmol/I NaCl, 1% IGEPAL^{®} CA-630, 0.5% sodium deoxycholate, 0.1% sodium dodecyl sulfate, 10% PhosSTOP Phosphatase Inhibitor Cocktail (Roche, Basel, Switzerland) and 10% cOmplete Mini Protease Inhibitor Cocktail (Roche). Lysates were centrifuged at 14850 x g for 15 min at 4 °C. Then, the total protein content of the lysates was determined using a bicinchoninic acid protein assay (ThermoFisher Scientific) in accordance with the manufacturer's instructions. The quantification of protein abundance was performed by Simple Western size-based assays using a 12-230 kDa and 66-440 kDa Separation Module (ProteinSimple, San Jose, CA, USA) according to the manufacturer's instructions. This fully automated Western blotting system is based on a capillary electrophoresis technology. The separation, immobilization to the capillary wall via a proprietary, photoactivated capture chemistry and immunodetection of the proteins using a horseradish peroxidase (HRP)-conjugated secondary antibody and chemiluminescent substrate take place directly in the capillary without gel separation and transfer steps to blotting paper. The chemiluminescence is measured at multiple exposure times and automatically quantified by the Compass for Simple Western software (version 4.1.0) (ProteinSimple). The chemiluminescent signal for immunodetected proteins, which can be represented as an electropherogram or virtual blot-like image, is automatically quantified by calculation of the area under the curve of the peak. The software reports the molecular weight, the area (in an arbitrary unit and in percent) and signal to noise ratio for each protein detected. The primary antibodies for the recognition of endogenous levels of total alpha-1 type 1 collagen (COL1A1) protein (#72026) and total fibronectin 1 (FN1) protein (#26836) were purchased from Cell Signaling Technology (Danvers, MA, USA). The anti-rabbit HRP-linked secondary antibody was from ProteinSimple. The antibodies for β-tubulin (#15115) Cell Signaling Technology) or vinculin (#13901) (Cell Signaling Technology) were used as endogenous loading controls to normalize relative protein expression of COL1A1 and FN1, respectively.

It was found that the sole treatment with HK1 or HK3 for 24 h had no impact on the protein abundance of COL1A1 and FN1. In contrast, TGF-β1 increased the protein levels of COL1A1 2.6 ± 0.2-fold (p<0.0001) and of FN1 3.0 ± 0.2-fold (p=0.0003) compared to untreated cells. After the treatment with 10 µM HK1 and 10 µM HK3 for 24h the upregulation of both fibrotic markers, COL1A1 and FN1, was completely suppressed in TGF-β1-treated LX2 cells.

Differences were analysed by one-way ANOVA followed by the Tukey method as correction for multiple testing. The results are shown in Fig. 1.

It was thus surprisingly found that HK1 and HK3 were able to revert the upregulation of the pro-fibrotic proteins alpha-1 type 1 collagen and fibronectin.

### Example 2: Prevention of the upregulation of proteins associated with NAFLD/NASH

LX2 cells were cultured and treated as described in Example 1. After the incubation time, the supernatants of LX2 cells were collected, centrifuged for 10 min at 2000 x g at 4 °C and transferred to new tubes to measure 92 proteins in the cell culture supernatants simultaneously using the Olink Inflammation multiplex immunoassay (Olink proteomics, Uppsala, Sweden). This panel covers 92 proteins, including pro- and anti-inflammatory cytokines, growth factors, chemokines and factors involved in biological processes such as apoptosis, inflammatory and immune response, cell adhesion, chemotaxis, or fibrosis. This protein quantification method is based on a proximity extension assay technology, which combines a dual-recognition immunoassay using two matched antibodies labelled with DNA oligonucleotides, with a polymerase chain reaction (PCR) amplification and a microfluidic quantitative real-time PCR as readout. The biomarker concentrations are given as normalized protein expression (NPX) values, which were generated from the threshold cycle values from the qPCR, and represent an arbitrary unit on a log2 scale.

It was found that 24 out of 92 proteins from this multimarker inflammation panel were detectable in the supernatants. The treatment with 5 ng/ml TGF-β1 as key driver of fibrosis led to increased secretion of leukaemia inhibitory factor (LIF) by 35.40 ± 9.76-fold (p=0.0013), vascular endothelial growth factor A (VEGFA) by 13.89 ± 2.40-fold (p=0.0004) (Fig. 2), and interleukin-6 (IL-6) by 4.99 ± 2.01-fold (p=0.0430) compared to untreated LX2 cells.

Surprisingly, it was found that HK1 and HK3 reduced the TGF-β1-induced secretion of LIF by more than 80% (82% to 84%, p-values 4.6 x 10-3 and 3.9 x 10-3) and VEGFA by more than 60% (63% to 65%, p-values 7.3 x 10-3 and 6.1 x 10-3) compared to TGF-β1-treated cells. Furthermore, the amount of secreted IL-6 was downregulated after the combined treatment with TGF-β1 and HK1 or HK3. Compared to TGF-treated LX2 cells, HK1 attenuated the protein content of IL-6 by 59%.

Differences were analysed by one-way ANOVA followed by the Tukey method as correction for multiple testing. The results are shown in Fig. 2.

### Example 3: Treatment of liver fibrosis in vivo

C57BL/6J mice were housed in housing cages GM500 (500 cm³) throughout the experimental phase. Animals' cages litters were changed at least once a week. They were housed in groups of 4 animals on a normal 12-hour light cycle (at 07:30 pm lights off), 22 ± 2 °C and 55 ± 10 % relative humidity. Animals were acclimated for 5 days. During the whole phase standard diet (SAFE, Ref.: U8400G10R) and tap water were provided ad libitum.

After the acclimation period, mice were intoxicated with CCl₄ i.p. twice weekly for 2 weeks to induce liver fibrosis. Body weight was recorded twice weekly for the 2-week intoxication period. After 2 weeks of CCl₄ injection (~72 hours after the 4^{th} injection of CCl₄), mice were weighted and bled at ~08:00am to collect blood (150µL blood on heparin by retro-orbital bleeding under isoflurane anaesthesia) and measure plasma ALT and AST levels. Mice were then randomized into 2 homogenous groups (n=10/group) based on 1) ALT, 2) AST and 3) body weight.

Mice were treated by oral gavage once daily with vehicle or HK3 for 2 weeks (from day 0 to day 13). During this 2-week treatment period, CCl₄ twice weekly i.p. injection was still performed until the end of the in-life phase. Body weight was recorded twice weekly, during the 2-week treatment period.

At the end of the treatment period (~48 hours after the last injection of CCl₄), all mice were weighed and 3h-fasted at ~10:00am. The last dose of vehicle and test compound was administered at ~10:00am, prior to blood collection (retro-orbital bleeding under isoflurane anesthesia for maximal volume/heparin) at ~1:00 pm. Plasma was isolated and immediately stored at -80°C.

Subsequently, the liver of each mouse was collected and weighted. Liver samples were dissected from the left lateral lobe for formalin-fixed paraffin-embedded samples for shipment for histology analysis (H&E and Sirius Red staining), % Sirius Red labelling and NAS scoring.

A total of 50 slides, each having a single hepatic lobe section stained with H&E (25) and Sirius red (25) were digitized using a Nanozoomer from Hamamatsu at Sciempath laboratory and posted on a secured digital slide server. They were downloaded and visualized using NDP viewer. Sections were evaluated with knowledge of groups but without knowledge of treatment. Each section was evaluated and individually scored.

A NAFLD scoring system (NAS), with which a total of four variables was qualitatively assessed and ranked with a score: (1) hepatocellular steatosis, (2) liver inflammation, (3) lobular fibrosis, and (4) hepatocyte ballooning. A detailed summary of criteria for score assignments are presented in the table below.

| **Score** | **Steatosis** | **Inflammation** | **Fibrosis** | **Hepatocyte ballooning** |
|---|---|---|---|---|
| **0** | < 5% of liver parenchyma | No foci | None | None |
| **1** | 5-to-33% of liver parenchyma | <2 foci at 20x field | Zone 3 and/or perisinusoidal fibrosis | Minimal to mild focal involving fewer than 3 hepatocytes per foci |
| **2** | 34-to-66% of liver parenchyma | 2-to-4 foci at 20x field | As grade 1 and portal fibrosis | Moderate multifocal involving more than 3 hepatocytes per foci |
| **3** | > 66% of liver parenchyma | > 4 foci at 20x field | As grade 2 and bridging fibrosis | Prominent multifocal involving large number of hepatocytes |
| **4** | Not applicable | Not applicable | Cirrhosis | Not applicable |

An individual mouse NAS total score was calculated for each animal by summing up the score for (1) hepatocellular steatosis, (2) liver inflammation, (3) lobular fibrosis, and (4) hepatocyte ballooning. Whole-section histomorphometric measurements of positive area for Sirius Red were performed using computer-assisted image analysis on digitized sections. The histomorphometric measurements were generated with the software from Visiopharm (Denmark) using an automated approach. Analysis was performed on virtual whole sections at 20x magnification for morphometric evaluation. The pixel value at 20x magnification corresponded to 0.46 µm/pixel.

An algorithm was prepared for Sirius Red (SR) morphometric measurement on liver-stained sections. The algorithm was generated with the Bayesian linear segmentation tool in the software package, which was further refined through training on a subset of sections from 5 animals. Major histology section artefacts, as well as, large vascular empty spaces were automatically dissected and removed from area of interest (AOI) or detected positive area. Also, positive collagen from capsule and large vessels media layer was automatically excluded from SR positive area.

The total area analysed or AOI (mm2), the total SR positive area (mm2) and the percent "SR" area / total liver area analysed (%) were determined. Each analysed image was individually verified for the accuracy of the morphometric evaluation.

It was found that the body weight and body weight gain were not altered by HK3 treatment, when compared to vehicle treatment. Furthermore, HK3 treatment did not change liver weight and relative liver weight as well as plasma ALT and AST levels when compared to vehicle treatment.

Surprisingly, a significant difference of fibrosis score was observed between the vehicle-treated group and the baseline group (p <0.05), which received a treatment with CCl₄ only for two weeks and were not treated by oral gavage once daily with vehicle or HK3 as described for the other groups above, whereas no significant difference was observed between the HK3-treated group and the baseline group. Likewise, a significant difference was observed between the vehicle-treated group and the HK3-treated group (p <0.05).

In the baseline group, all mice had a fibrosis score of 2. In the vehicle group, 6 mice had a fibrosis score equal to 3 and 4 mice had a fibrosis score equal to 2. In the HK3-treated group, 1 mouse had a fibrosis score equal to 3 and 9 mice had a fibrosis score equal to 2.

The following table shows the scores obtained for the respective animals for the respective parameter.

| | **Steatosis** | **Inflammation** | **Fibrosis** | **Hepatocyte ballooning** |
|---|---|---|---|---|
| **Vehicle** | 1 | 1 | 3 | 0 |
| | 1 | 1 | 3 | 0 |
| | 1 | 1 | 3 | 1 |
| | 1 | 1 | 3 | 0 |
| | 1 | 1 | 2 | 0 |
| | 1 | 1 | 2 | 0 |
| | 1 | 1 | 2 | 0 |
| | 1 | 1 | 3 | 1 |
| | 1 | 1 | 2 | 0 |
| | 1 | 1 | 3 | 1 |
| **HK3** | 1 | 1 | 2 | 1 |
| | 1 | 1 | 3 | 0 |
| | 1 | 1 | 2 | 1 |
| | 1 | 1 | 2 | 0 |
| | 1 | 1 | 2 | 0 |
| | 1 | 1 | 2 | 0 |
| | 1 | 1 | 2 | 0 |
| | 1 | 1 | 2 | 0 |
| | 1 | 1 | 2 | 1 |
| | 1 | 1 | 2 | 0 |
| **Baseline** | 0 | 0 | 2 | 0 |
| | 0 | 0 | 2 | 0 |
| | 0 | 0 | 2 | 0 |
| | 0 | 0 | 2 | 0 |
| | 0 | 0 | 2 | 0 |

These findings demonstrate that HK3 treatment reduced / prevented CCl₄-induced hepatic fibrosis process.

Furthermore, it was found that the percentage of Sirius Red labelling was significantly increased in vehicle (p <0.001) and HK3-tread (p <0.01) groups when compared to baseline group, which did not receive CCl₄. However, HK3-treatment led to a significantly reduced percentage of Sirius Red labelling compared to the vehicle group (p<0.05).

These findings demonstrate that HK3 treatment can reduce hepatic collagen content in the CCl₄-intoxicated mouse model, supporting that HK3 treatment can reduce the amount of collagen in hepatic fibrosis.

Moreover, it was found that HK3 caused a slight but significant downregulation of TNF-α levels in liver homogenates after combined administration of HK3 and CCl₄ compared to CCl₄-treated group (p<0.05).

Statistical analysis was performed using a Two-way ANOVA + Bonferroni's post-test (body weight follow-up), a 1-way ANOVA + Dunnett's post-test or if significant variance was found, a Kruskal-Wallis with Dunn's post-test. When relevant, one single treatment group was compared with the vehicle group using an unpaired, 2-tailed, Student t-test. A p<0.05 was defined as significant.

The results are shown in Figs. 3 and 4.

### Example 4: Hepatoprotective effect of HK1 and HK3

The potential protective effect of HK1 and HK3 on caspase 3/7 activity in HepG2 cells and primary human hepatocytes was analysed.

HepG2 cells were obtained from Merck (Darmstadt, Germany) (ECACC-certified), while primary human hepatocytes were obtained from Thermo Fisher Scientific (Waltham, MA, USA), and all were cultured according to the instructions of the respective manufacturers. During experiments, cells were cultured in serum-free medium. Unless otherwise stated, cells were treated with 200 µM bovine serum albumin (BSA)-conjugated palmitate (Cayman Chemicals, Ann Arbor, MI) alone or in combination with 10 µM HK1 (Taros Chemicals GmbH & Co. KG, Dortmund, Germany) and HK3 (Taros Chemicals GmbH & Co. KG, Dortmund, Germany) 30 min prior to palmitate exposure for 24 hours. The vehicle BSA was used according to the palmitate concentration and DMSO according to HK1 and HK3 concentration. 2 µM Staurosporine (Abcam, Cambridge, GB) was used as a positive and 100 µM Caspase 3/7 inhibitor (CI) (Cayman Chemicals) as a negative control.

It was surprisingly found that pre-treatment with HK1 and HK3 (10 µM) prevents caspase 3/7 activity provoked by palmitate (200 µM) (262.6% ± 38.32% and 42.28% ± 8.28% vs. 428.8% ± 62.2% of control, respectively, p≤0.01 and p≤0.0001).

As a negative control, the proapoptotic effect of palmitate was abolished in the presence of the caspase 3/7 inhibitor CI (29.65% ± 24.56% vs. 428.8% ± 62.2%). Staurosporine (STP) was used as a positive control to induce caspase 3/7 activity (687.2% ± 132.4%).

The preventive effect of HK1 and HK3 on apoptosis has been confirmed in primary human hepatocytes. Pre-treatment with HK1 and HK3 (10 µM) completely prevents the caspase 3/7 activity provoked by palmitate (200 µM) (85.57% ± 9.51%, 41.73% ± 5.33% respectively vs. 128.7% ± 6.05% of control, P ≤ 0.001, P ≤ 0.0001). Caspase 3/7 inhibitor (100 µM) reduced caspase 3/7 activity in combination with palmitate (200 µM) to 21.23% ± 4.33%.

Differences were analysed by one-way ANOVA. The results are shown in Fig. 5.

Surprisingly, this effect was not obtained by many other thioacrylamide derivatives tested, or was much weaker than the effect provided by HK1 and HK3.

### Example 5: Hepatoprotective effect of different thioacrylamide derivatives

A hepatoprotective effect was determined as described in Example 4. A treatment was performed with HK1, HK2, HK3, HK4 or HK5 (100 nM) either alone or in combination with palmitate (200 µM).

It was surprisingly found that HK1 and HK3 provided a stronger reduction of the caspase 3/7 activity provoked by palmitate than other thioacrylamide derivatives, particularly than HK2 or HK4. It was further surprisingly found that HK3 provided the strongest reduction of the caspase 3/7 activity provoked by palmitate among all tested thioacrylamide derivatives.

Differences were analysed by one-way ANOVA, n=4. The results are shown in Fig. 6.

### Example 6: Viability, reduced lipid accumulation, cytokine secretion and pro-collagen concentration in NASH patient-derived 3D spheroid model

Experiments were conducted using HepaPredict's (HepaPredict AB, Sweden) 3D primary human hepatocyte (PHH) spheroid NASH model (e.g. as published in Bell et al. Sci Rep. 2016 May 4;6:25187. doi: 10.1038/srep25187).

Cryopreserved primary human hepatocytes were obtained from BiolVT (USA) and nonparenchymal cells (NPC) from LifeNetHealth (USA). After thawing, PHH-NPC cocultures were seeded in a multi-well 96-well Elplasia plate (Corning) in William's E medium containing 11 mM glucose, 10 µg/mL (control) or 10 mg/mL insulin (NASH), 100 nM dexamethasone, 5.5 mg/L transferrin, 6.7 µg/L selenite, 2 mM L-glutamine, 1 U/mL penicillin, 0.1 mg/mL streptomycin, and 10% fetal bovine serum (FBS). PHH-NPC cocultures were seeded in ratio of 6:1 PHH:NPC with a total of 40,000 PHH per well. When spheroids were formed, FBS was phased out and spheroids were maintained in either the same media to provide the negative control ("Ctrl"), or with addition of albumin-conjugated free fatty acid (FFA), comprised of 240 µM palmitic acid and 240 µM oleic acid (NASH medium) to provide the positive control ("NASH"). Compound exposure was conducted in NASH medium with medium exchanges every 48-72 hours.

For cell viability and intracellular lipid accumulation assays, cells were seeded as previously reported (Bell et al., SciRep, 2016). In short, PHH and NPC were seeded as a single spheroid per well into 96-well ultra-low attachment (ULA) plates (Corning) at 1,500 viable cells per well with the PHH:NPC ratio 6:1.

Characteristics of the utilized human donors are provided in the below table.

| | **PHH** | **NPC** |
|---|---|---|
| **Age** | 30 | 45 |
| **Ethnicity** | Hispanic | Hispanic |
| **Gender** | Female | Female |
| **BMI** | 30.8 | 32 |
| **Cause of death** | Head trauma | Anoxia |
| **HEP-B, HEP-C, HIV** | Negative | Negative |
| **Medications** | None reported | None reported |

After spheroid formation, cells were exposed to up to five different concentrations of the candidate molecules HK1 or HK3 (0.1 µM, 0.3 µM, 1 µM, 3 µM, and 10 µM) with or without FFA in the culture medium. All compound stocks and dilutions were prepared in DMSO, aliquoted and stored protected from light at -20°C. Fresh dosing solutions were prepared from these aliquots on each dosing day. All endpoints were evaluated after 14 days of exposure.

For statistical comparisons, normalized values for a given condition were compared to either the corresponding vehicle (DMSO) controls or the respective FFA (NASH) controls at the same time point using heteroscedastic unpaired two-tailed t-tests.

The experimental data was judged to be of sufficient quality if control spheroids treated with vehicle showed ATP levels comparable to previous experiments using hepatocytes from the same donor. Only wells that contained spheroids after visual inspection at the time of sampling were included in the analysis, as previously reported (Vorrink et al., ToxSci, 2018).

### Example 6.1: Cell viability

Viability was evaluated by ATP quantification using the CellTiter-Glo Luminescent Cell Viability Assay (Promega) according to the manufacturer's instructions. Briefly, individual spheroids were disrupted in 25 µL of the supplied buffer followed by incubation at 37°C in 5% CO2 for 20 minutes. Subsequently, luminescence signals were measured using a SpectraMax iD5 multi-mode microplate reader (Molecular Devices). Viability was evaluated after up to two weeks of exposure and values are plotted as relative to vehicle (DMSO) controls with or without FFA. Up to eight spheroids were measured per endpoint.

The in vitro hepatotoxic potential of the two candidate compounds HK1 and HK3 was evaluated after 48 hours, 1 week, and 2 weeks by comparing the viability of spheroids in the respective treatment conditions to the corresponding vehicle (DMSO) control.

It was found that concentrations of from 0.1 µM to 10 µM did not affect the viability of the spheroids even when treated for 2 weeks.

### Example 6.2: Intracellular lipid accumulation

Intracellular lipid accumulation was quantified using the AdipoRed Adipogenesis Assay Kit (Lonza) following the manufacturer's instructions. Briefly, 3.5 µL of AdipoRed Assay Reagent was added to trypsinized single spheroids followed by 10-15 minutes incubation in the dark. Afterwards, spheroids were disrupted by vigorous pipetting and fluorescence was measured (Ex/Em: 485/572) using a SpectraMax iD5 multi-mode microplate reader (Molecular Devices).

To evaluate whether the two candidate compounds HK1 and HK3 impacted hepatic steatosis, we assessed their impact on intracellular triglyceride accumulation after 1 week of exposure.

Spheroids were treated with either 1 µM, 3 µM or 10 µM HK1 or HK3.

In the positive control ("NASH") steatosis was highly induced (considered 100%). We observed significant decreases in hepatic lipid contents after both HK1 and HK3 exposure across the tested concentration range of 1 µM to 10 µM. Lipid contents of spheroids exposed to HK1 were reduced to 79% (1 µM), 81% (3 µM), and 62% (10 µM) compared to NASH. Exposure to HK3 resulted in even less accumulation of intracellular lipids reaching only between 46-49% compared to the positive control. Interestingly, HK3 also showed less variability in the ability to reduce lipid accumulation. In summary, both HK1 and HK3 were found to significantly decrease intracellular lipid levels in 3D PHH:NPC spheroids. The results are shown in Fig. 7.

Surprisingly, HK3 provided a stronger effect than HK1.

### Example 6.3: Cytokine secretion

Secreted cytokines (IL-6 and IL-8) were measured in culture supernatants using a customized Magnetic Luminex Performance Assay (Human Cytokine Panel A, R&D Systems). The multiplexed assay was performed following the manufacturer's instructions. Standard curves were generated by resuspending the human recombinant cytokines mixture at concentrations ranging from 1 to 4000 pg/ml with 8 points at 1:3 series dilution for the customized selection of 3 analytes, IL-6 and IL-8/CXCL8. Cell culture supernatants were diluted 2 to 10 times and a total volume of 50 µl/well was used. Standards/samples were well mixed with the beads and incubated for 3 hours at room temperature (RT) on an orbital shaker at 800 rpm in the dark. Afterwards, the plates were washed three times with a magnetic plate washer, then biotinylated detection antibodies were added to the plates followed by an incubation for 1 hour at RT; after three times wash, the plates were incubated with PE-conjugated streptavidin solution for 30 minutes at RT. The fluorescence intensity was measured using a Bio-Plex 200 system (Bio-Rad), the median fluorescence intensity of each analyte was analyzed with the Bio-Plex Manage software (version 6.2) using a 5-parameter regression algorithm. All bead counts were checked and any analyte with counts below 40 were excluded from further analysis. The accepted recovery range of the standard curves was between 80% to 120%, any points out of the range were excluded from the analysis.

To assess the effect of the two candidate compounds HK1 and HK3 on hepatic inflammation, we measured levels of the pro-inflammatory cytokines IL-6 and IL-8.

NASH spheroids showed slightly elevated levels of IL-6 and IL-8. Upon treatment, HK3 exposure led to a slight decrease in both IL-6 and IL-8 secretion.

In contrast, HK3 exposure led to a stronger decrease in both IL-6 and IL-8 secretion. Exposure to 1-10 µM HK3 resulted in a reduction of IL-6 levels to 73%, 55%, and 72% compared to NASH, respectively. IL-8 levels reached between 80-91% compared to NASH. The results are shown in Fig. 8.

### Example 6.4: Fibrosis assessment

Supernatant from each well was collected and stored at -80C. On the day of the measurement, samples were thawed and fibrosis was evaluated using the Human Pro-collagen 1 alpha 1 DuoSet ELISA (R&D Systems) where pro-collagen 1a1 levels were measured in culture supernatants. In short, samples were diluted 1:50 using reagent diluent, incubated with mouse anti-human pro-collagen 1a1 and, subsequently with biotinylated sheep anti-human pro-collagen 1a1 as the detection antibody. The assay was performed according to the manufacturer's instructions.

Compared to the NASH control, particularly the treatment with HK3 resulted in significant reduction of pro-collagen 1a1 levels. Exposure with HK3 was dose-dependent and reached levels of 82%, 73%, and 64% compared to NASH, respectively. The results are shown in Fig. 9.

## Claims

1. Thioacrylamide derivative for use in the prevention or treatment of liver fibrosis in a subject,
wherein the thioacrylamide derivative is selected from the group consisting of 2-Cyano-3-cyclopropyl-N-(4-fluoro-3-methyl-phenyl)-3-hydroxy-thioacrylamide (i.e. HK1), 2-Cyano-3-cyclopropyl-3-hydroxy-N-(3-methyl-4-nitro-phenyl)-thioacrylamide (i.e. HK3), a tautomer of HK1, a tautomer of HK3, or mixtures thereof, or pharmaceutically acceptable salts thereof or solvates thereof,
wherein the liver fibrosis is caused by or resulting from non-alcoholic fatty liver disease (NAFLD).

2. Pharmaceutical composition comprising a thioacrylamide derivative as defined in claim 1 for use in the prevention or treatment of liver fibrosis in a subject,
wherein the liver fibrosis is caused by or resulting from non-alcoholic fatty liver disease (NAFLD).

3. Thioacrylamide derivative or pharmaceutical composition for use according to one of the preceding claims, wherein the liver fibrosis is caused by or **characterized by** or includes the activation of hepatic stellate cells.

4. Thioacrylamide derivative or pharmaceutical composition for use according to one of the preceding claims, wherein the liver fibrosis is caused by or **characterized by** or includes increased amounts of alpha-1 type 1 collagen and/or fibronectin in the hepatic tissue of the subject.

5. Thioacrylamide derivative or pharmaceutical composition for use according to one of the preceding claims, wherein the liver fibrosis is caused by or **characterized by** or includes increased amounts of one, two, three or all proteins selected from the group consisting of leukaemia inhibitory factor (LIF), vascular endothelial growth factor A (VEGFA), Tumor necrosis factor α (TNF-α), interleukin-6 (IL-6) and interleukin-8 (IL-8) in the blood, preferably in the blood serum, of the subject.

6. Thioacrylamide derivative or pharmaceutical composition for use according to claim 4 or 5, wherein the increased amounts of the, one, two, three or more or all proteins are caused by increased protein amounts of transforming growth factor β1 (TGF-β1),
preferably in the hepatic tissue of the subject,
preferably wherein TGF-β1 refers to activated TGF-β1.

7. Thioacrylamide derivative or pharmaceutical composition for use according to one of the preceding claims, wherein the prevention or treatment of liver fibrosis is or includes the prevention or treatment of liver cirrhosis.

8. Thioacrylamide derivative or pharmaceutical composition for use according to one of the preceding claims, wherein the prevention or treatment comprises the administration of at least 5 mg/kg body weight of the subject, preferably at least 7.5 mg/kg, preferably at least 10 mg/kg, of HK1, of HK3 or of a combination of HK1 and HK3.

## Patentansprüche

1. Thioacrylamidderivat zur Anwendung in der Vorbeugung oder Behandlung von Leberfibrose in einem Subjekt,
wobei das Thioacrylamidderivat ausgewählt ist aus der Gruppe bestehend aus Cyano-3-cyclopropyl-N-(4-fluoro-3-methyl-phenyl)-3-hydroxy-thioacrylamid (d.h. HK1), 2-Cyano-3-cyclopropyl-3-hydroxy-N-(3-methyl-4-nitro-phenyl)-thioacrylamid (d.h. HK3), einem Tautomer von HK1, einem Tautomer von HK3, oder Mischungen davon, oder pharmazeutisch akzeptable Salze davon oder Solvate davon,
wobei die Leberfibrose durch die nichtalkoholische Fettlebererkrankung (NAFLD) ausgelöst ist oder sich daraus ergibt.

2. Pharmazeutische Zusammensetzung enthaltend ein Thioacrylamidderivat wie in Anspruch 1 definiert zur Anwendung in der Vorbeugung oder Behandlung von Leberfibrose in einem Subjekt,
wobei die Leberfibrose durch die nichtalkoholische Fettlebererkrankung (NAFLD) ausgelöst ist oder sich daraus ergibt.

3. Thioacrylamidderivat oder pharmazeutische Zusammensetzung zur Anwendung nach einem der vorangehenden Ansprüche, wobei die Leberfibrose durch die Aktivierung von Leber-Sternzellen ausgelöst ist, **dadurch gekennzeichnet** ist oder diese enthält.

4. Thioacrylamidderivat oder pharmazeutische Zusammensetzung zur Anwendung nach einem der vorangehenden Ansprüche, wobei die Leberfibrose durch erhöhte Mengen an alpha-1 Typ-1 Kollagen und/oder Fibronektin in Lebergewebe des Subjekts ausgelöst ist, **dadurch gekennzeichnet** ist oder diese enthält.

5. Thioacrylamidderivat oder pharmazeutische Zusammensetzung zur Anwendung nach einem der vorangehenden Ansprüche, wobei die Leberfibrose durch erhöhte Mengen an einem, zwei, drei oder allen Proteinen ausgewählt aus der Gruppe bestehend aus Leukämiehemmender Faktor (LIF), vaskulärer endothelialer Wachstumsfaktor A (VEGF-A), Tumornekrosefaktor a (TNF-α), Interleukin-6 (IL-6) und Interleukin-8 (IL-8) im Blut, vorzugsweise im Blutserum, des Subjekts ausgelöst ist, **dadurch gekennzeichnet** ist oder diese enthält.

6. Thioacrylamidderivat oder pharmazeutische Zusammensetzung zur Anwendung nach Anspruch 4 oder 5, wobei die erhöhten Mengen des, eines, zwei, drei oder mehrerer oder aller Proteine durch erhöhte Proteinmengen von Transforming Growth Factor β1 (TGFβ1) ausgelöst sind,
vorzugsweise im Lebergewebe des Subjekts,
vorzugsweise wobei sich TGFβ1 auf aktiviertes TGFβ1 bezieht.

7. Thioacrylamidderivat oder pharmazeutische Zusammensetzung zur Anwendung nach einem der vorangehenden Ansprüche, wobei die Vorbeugung oder Behandlung der Leberfibrose die Vorbeugung oder Behandlung der Leberzirrhose enthält oder ist.

8. Thioacrylamidderivat oder pharmazeutische Zusammensetzung zur Anwendung nach einem der vorangehenden Ansprüche, wobei die Vorbeugung oder Behandlung die Verabreichung von mindestens 5 mg/kg Körpergewicht des Subjekts, vorzugsweise mindestens 7,5 mg/kg, vorzugsweise mindestens 10 mg/kg, an HK1, an HK3 oder an einer Kombination an HK1 und HK3 umfasst.

## Revendications

1. Dérivé de thioacrylamide destiné à être utilisé dans la prévention ou le traitement de la fibrose hépatique chez un sujet,
dans lequel le dérivé de thioacrylamide est choisi dans le groupe constitué par le 2-cyano-3-cyclopropyl-N-(4-fluoro-3-méthyl-phényl)-3-hydroxy-thioacrylamide (c'est-à-dire HK1), le 2-cyano-3-cyclopropyl-3-hydroxy-N-(3-méthyl-4-nitro-phényl)-thioacrylamide (c'est-à-dire HK3), un tautomère de HK1, un tautomère de HK3, ou des mélanges de ceux-ci, ou des sels pharmaceutiquement acceptables de ceux-ci ou des solvates de ceux-ci,
dans lequel la fibrose hépatique est provoquée par ou résulte d'une stéatose hépatique non alcoolique (NAFLD).

2. Composition pharmaceutique comprenant un dérivé de thioacrylamide tel que défini dans la revendication 1, destinée à être utilisée dans la prévention ou le traitement de la fibrose hépatique chez un sujet,
dans lequel la fibrose hépatique est provoquée par ou résulte d'une stéatose hépatique non alcoolique (NAFLD).

3. Dérivé de thioacrylamide ou composition pharmaceutique destinée à être utilisée selon l'une quelconque des revendications précédentes, dans lequel la fibrose hépatique est provoquée par ou **caractérisée par** ou comprend l'activation de cellules stellaires hépatiques.

4. Dérivé de thioacrylamide ou composition pharmaceutique destiné(e) à être utilisé(e) selon l'une quelconque des revendications précédentes, dans lequel la fibrose hépatique est provoquée par ou **caractérisée par** ou comprend des quantités accrues de collagène alpha-1 de type 1 et/ou de fibronectine dans le tissu hépatique du sujet.

5. Dérivé de thioacrylamide ou composition pharmaceutique destiné(e) à être utilisé(e) selon l'une quelconque des revendications précédentes, dans lequel la fibrose hépatique est provoquée par ou **caractérisée par** ou comprend des quantités accrues d'une, de deux, de trois ou de toutes les protéines choisies dans le groupe constitué par le facteur inhibiteur de la leucémie (LIF), le facteur de croissance endothélial vasculaire A (VEGFA), le facteur de nécrose tumorale a (TNF-α), l'interleukine-6 (IL-6) et l'interleukine-8 (IL-8) dans le sang, de préférence dans le sérum sanguin, du sujet.

6. Dérivé de thioacrylamide ou composition pharmaceutique destiné(e) à être utilisé(e) selon la revendication 4 ou 5, dans lequel les quantités accrues de la, d'une, de deux, trois ou plusieurs ou toutes les protéines sont provoquées par des quantités accrues de protéines du facteur de croissance transformant β1 (TGF-β1),
de préférence dans le tissu hépatique du sujet,
de préférence dans lequel TGF-β1 se réfère à du TGF-β1 activé.

7. Dérivé de thioacrylamide ou composition pharmaceutique destiné(e) à être utilisé(e) selon l'une quelconque des revendications précédentes, dans lequel la prévention ou le traitement de la fibrose hépatique est ou comprend la prévention ou le traitement de la cirrhose hépatique.

8. Dérivé de thioacrylamide ou composition pharmaceutique destiné(e) à être utilisé(e) selon l'une quelconque des revendications précédentes, dans lequel la prévention ou le traitement comprend l'administration d'au moins 5 mg/kg de poids corporel du sujet, de préférence d'au moins 7,5 mg/kg, de préférence d'au moins 10 mg/kg, de HK1, de HK3 ou d'une combinaison de HK1 et de HK3.
